# FASCICULE DE BREVET EUROPEEN

(11) **EP 2 150 267 B1**
(45) Date de publication et mention de la délivrance du brevet: **20.03.2013**
(21) Numéro de dépôt: 08805497.8
(22) Date de dépôt: 23.04.2008
(51) Int. Cl.: C07K 5/08, C07K 5/10, A61K 38/08, A61K 8/64, A61K 38/45, A61P 17/00

(54) **COMPOSITION PHARMACEUTIQUE ET/OU COSMETIQUE CONTENANT UN PRINCIPE ACTIF ACTIVATEUR DU CYTOCHROME C**
PHARMAZEUTISCHE UND/ODER KOSMETISCHE ZUSAMMENSETZUNG MIT EINEM CYTOCHROM-C-AKTIVIERENDEM WIRKSTOFF
PHARMACEUTICAL AND/OR COSMETIC COMPOSITION CONTAINING A CYTOCHROME C-ACTIVATING ACTIVE INGREDIENT

(30) Priorité: 27.04.2007 FR 0703057
(43) Date de publication de la demande: 10.02.2010
(73) Titulaire: Societe D'extraction Des Principes Actifs S.a. (ISP Vincience), 06904 Sophia-Antipolis Cedex (FR)
(72) Inventeur: DAL FARRA, Claude, F-06650 Opio (FR); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2008/000578
(87) Numéro de publication internationale: WO 2008/145855

(56) Documents cités:
- EP-A- 1 864 996
- WO-A-00/63236
- WO-A-02/08752
- WO-A-02/26254
- WO-A-02/46416
- WO-A-2004/043482
- WO-A-2005/097060
- WO-A-2007/058625
- FRANKEL, MAX ET AL: "Synthesis of peptides related to the C terminus of horse heart cytochrome c" COLLOQUES INTERNATIONAUX DU CENTRE NATIONAL DE LA RECHERCHE SCIENTIFIQUE , NO. 175, 278-82 CODEN: COINAV; ISSN: 0366-7634, 1968, XP009093273
- GONDRAN C ET AL: "A new synthetic peptide that exhibits interesting anti-aging effects" JOURNAL OF INVESTIGATIVE DERMATOLOGY, vol. 126, no. Suppl. 1, avril 2006 (2006-04), page 27, XP009093186 & 67TH ANNUAL MEETING OF THE SOCIETY-FOR-INVESTIGATIVE-DERMATOLOGY; PHILADELPHIA, PA, USA; MAY 03 -06, 2006 ISSN: 0022-202X
- DEL FARRA ET AL.: "Anti-aging effects observed in new synthetic peptide" J AM ACAD DERMATOL, février 2007 (2007-02), page AB25, XP002460912
- "An anti-aging effect on the lips and skin observed in in vivo studies on a new fibronectin-like peptide" J AM ACAD DERMATOL, février 2007 (2007-02), page AB88, XP002460913

### SEQUENCE LISTING

<110> Société d'Extraction des Principes Actifs (ISP VINCIENCE)
<120> COMPOSITION PHARMACEUTIQUE ET/OU COSMETIQUE CONTENANT UN PRINCIPE ACTIF ACTIVATEUR DU CYTOCHROME C
<130> Bv PCT 07-91
<150> FR0703057
   <151> 2007-04-27
<160> 4
<170> PatentIn version 3.3
<210> 1
   <211> 5
   <212> PRT
   <213> Artificial
<220>
   <223> Synthetic Peptide
<400> 1
   Tyr Leu Lys Lys Ala
   1               5
<210> 2
   <211> 5
   <212> PRT
   <213> Artificial
<220>
   <223> Synthetic Peptide
<220>
   <221> MOD_RES
   <222> (5)..(5)
   <223> AMIDATION
<400> 2
   Tyr Leu Lys Lys Ala
   1               5
<210> 3
   <211> 4
   <212> PRT
   <213> Artificial
<220>
   <223> Synthetic Peptide
<220>
   <221> MOD_RES
   <222> (4)..(4)
   <223> AMIDATION
<400> 3
   Tyr Leu Lys Lys
   1
<210> 4
   <211> 3
   <212> PRT
   <213> Artificial
<220>
   <223> Synthetic Peptide
<400> 4
   Tyr Leu Lys
   1

## Description

La présente invention se situe dans le domaine cosmétique et pharmaceutique, et plus particulièrement dans le domaine de la dermatologie. La présente invention concerne une composition cosmétique ou pharmaceutique, et notamment dermatologique, comprenant, dans un milieu physiologiquement adapté, un principe actif capable d'activer le cytochrome c. Ce principe actif est constitué d'au moins un peptide, utilisé seul ou en association avec au moins un autre principe actif. L'utilisation d'une composition cosmétique destinée à stimuler les fonctions des mitochondries et à augmenter le niveau énergétique cellulaire est également décrite. Un procédé de traitement cosmétique destiné à protéger la peau et les phanères des agressions extérieures et à lutter contre le vieillissement cutané est également décrit.

Le principe actif peut également être utilisé pour préparer des compositions pharmaceutiques destinées à prévenir ou lutter contre les pathologies liées à des dysfonctionnements mitochondriaux ; par exemple, certaines dégénérescences neuromusculaires ou cardiaques, le diabète de type II ou encore certaines pathologies du vieillissement.

Le terme « phanères » englobe l'ensemble des annexes kératiniques présentes à la surface du corps, en particulier les poils, les cils, les sourcils, les ongles et les cheveux.

La peau est un organe vital qui recouvre toute la surface du corps et assure des fonctions protectrices, sensitives, immunitaires, métaboliques ou encore thermorégulatrices. La peau, comme les autres organes, est soumise au vieillissement. Or, un des mécanismes majeurs impliqués dans les processus du vieillissement est l'accumulation de dommages oxydatifs dans des molécules essentielles telles que les lipides membranaires, les protéines, l'ADN et tout particulièrement l'ADN mitochondrial (ADNmt).

Les dommages oxydatifs sont provoqués par les radicaux libres, des espèces chimiquement instables et très réactionnelles générées par le métabolisme intracellulaire ou les agressions extérieures. Parmi ces agressions extérieures, on peut citer : les rayonnements UV, les toxines, les polluants atmosphériques, les oxydants alimentaires. Dans la peau, on observe un vieillissement prématuré survenant dans les zones exposées aux rayonnements, caractérisé par des phénomènes d'altérations des macromolécules (péroxydation lipidique, carbonylation des protéines) touchant en particulier l'élastine, le collagène ou la fibronectine. On a également pu montrer un déclin progressif des fonctions mitochondriales avec l'âge, probablement lié à l'accumulation de mutations sur l'ADNmt (K. Singh, Ann. N.Y. Acad. Sci. 1019, 2004).

Une des conséquences importantes de l'accumulation des dommages oxydatifs est une réduction de la capacité de la cellule à produire de l'ATP (Porteous et al., Eur J Biochem 1998, 257(1): 192-201). Ainsi, le phénomène du vieillissement cellulaire est en relation avec les dommages oxydatifs que subit la cellule mais aussi avec le processus de production d'énergie nécessaire à la cellule pour survivre.

L'organisme possède des mécanismes de défense capables de piéger ou de transformer les radicaux libres (enzymes, glutathion, vitamines A et E, coenzyme Q10, etc.). Cependant, ces systèmes de défense antioxydants s'avèrent souvent insuffisants devant les nombreux stress et agressions extérieures auxquels sont soumis les organismes et la peau en particulier.

Dans ce contexte, les propriétés particulières du cytochrome c apparaissent comme particulièrement intéressantes :

Le cytochrome c est une petite protéine soluble de 15 kDa qui joue un rôle essentiel dans la fonction mitochondriale et dans la survie cellulaire. Le cytochrome c est une molécule hautement conservée chez la plupart des eucaryotes ; on la trouve dans les mitochondries des plantes, des animaux et de nombreux organismes unicellulaires. Le cytochrome c présente une structure protéique organisée autour d'une porphyrine, constituée de quatre noyaux pyrrol, eux-mêmes liés à un atome de fer.

Le rôle principal du cytochrome c est d'assurer le transfert d'électrons, grâce au changement de valence de l'atome de fer. Le cytochrome c, qui est soluble, transporte ainsi les électrons depuis le complexe III (coenzyme QH2-cytochrome c réductase) jusqu'au complexe IV (cytochrome oxydase). Les électrons, qui sont le substrat de la cytochrome oxydase, sont alors transférés par l'enzyme vers l'oxygène.

La recherche de composés pouvant stimuler les mitochondries et augmenter le niveau énergétique cellulaire afin de prévenir ou de lutter contre les signes du vieillissement cutané, ou les dommages causés par les agressions extérieures, telles que les rayonnements UV, les radiations, ou les expositions à des toxines ou à des polluants, est une préoccupation importante de la recherche médicale et de la cosmétique. A cet égard, il a été proposé des solutions telles que l'apport de substances impliquées dans le métabolisme énergétique, et plus particulièrement d'intermédiaires ou de cofacteurs du cycle de Krebs tels que le fumarate, le L-malate, l'acétyl CoA (WO 02064129), ou encore le traitement de la peau par des substances capables de diminuer les radicaux libres, telles que la vitamine C (US 2004/0086526) ou la L-ergothionéine (WO 9836748). On connaît également les documents EP1864996, WO0246416, WO0063236 et WO2007058625 qui décrivent des compositions comprenant des peptides répondant à la formule générale (I), ainsi que leur utilisation thérapeutique. Mais à la connaissance de la demanderesse, aucune composition cosmétique ou pharmaceutique comprenant des peptides répondant à la formule générale (i), capables d'activer le cytochrome c ni l'utilisation de tels peptides dans le domaine cosmétique ou dermatologique n'ont encore été décrites.

Les inventeurs ont en effet mis en évidence une activité cosmétique et dermatologique, de certains peptides répondant à la formule générale (i), capables d'activer le cytochrome c. Il a notamment été mis en évidence que ces peptides, lorsqu'ils sont appliqués sur la peau, stimulent de façon importante les fonctions mitochondriales. Cela a, entre autre, été démontré par une augmentation de l'expression du cytochrome c et une augmentation de la synthèse d'ATP. Ce nouveau principe actif stimulateur des mitochondries et plus généralement capable de protéger la peau des agressions extérieures, permet ainsi d'ouvrir de nouvelles perspectives thérapeutiques et cosmétiques.

On entend par «principe actif capable de protéger la peau des agressions extérieures », toute substance capable de présenter des propriétés protectrices ou de diminuer l'apoptose dans des cellules ou des tissus soumis à un stress d'origine physicochimique ou environnementale.

On entend par « principe actif capable d'activer le cytochrome c », toute substance capable d'augmenter l'expression du cytochrome c, soit par l'activation de la synthèse protéique (par modulation directe ou indirecte de l'expression génique du cytochrome c), soit par l'augmentation de l'activité biologique du cytochrome c, soit par d'autres processus biologiques tels que la stabilisation de la protéine cytochrome c ou encore la stabilisation des transcrits d'ARN messager.

Les substances capables d'activer le cytochrome c selon l'invention sont des peptides.

Ainsi, l'invention a pour objet premier une composition cosmétique ou dermatologique, comprenant, dans un milieu physiologiquement adapté, des peptides capables d'activer le cytochrome c. en tant que principe actif, seul ou en association avec au moins un autre principe actif, dont le nombre d'acides aminés est compris entre 3 et 13, et qui
possède une séquence qui répond à la formule générale (I)

R₁-(AA)ₙ- X₁-X₂- X₃- X₄-X₅- (AA)ₚ-R₂

Dans laquelle
X₁ est la tyrosine,
X₂ est la leucine,
X₃ est la lysine,
X₄ est la thréonine, la sérine, la lysine, l'arginine ou aucun acide aminé,
X₅ est la valine, l'alanine, la tyrosine, la méthionine ou aucun acide aminé,
AA représente un acide aminé quelconque, ou un de ses dérivés, et n et p sont des nombres entiers compris entre 0 et 4.
R₁ représente la fonction amine primaire de l'acide aminé N-terminal, libre ou substitué par un groupement protecteur pouvant être choisi parmi un groupement acétyle, un groupement benzoyle, un groupement tosyle ou un groupement benzyloxycarbonyle.
R₂ représente la fonction hydroxyle de l'acide carboxyle de l'acide aminé C-terminal, libre ou substitué par un groupement protecteur pouvant être choisi parmi une chaîne alkyle de C₁ à C₂₀, ou un groupement NH₂. NHY ou NYY avec Y représentant une chaîne alkyle de C₁ à C₄, correspondant à une des séquences suivantes :
(SEQ ID n°1) Tyr-Leu-Lys-Lys-Ala
(SEQ ID n°2) Tyr-Leu-Lys-Lys-Ala -NH₂
(SEQ ID n°3) Tyr-Leu-Lys-Lys-NH₂
(SEQ ID n°4) Tyr-Leu-Lys

Selon un mode de réalisation particulièrement intéressant, le peptide biologiquement actif correspond à la séquence SEQ ID n°1.

Selon un autre mode de réalisation particulièrement intéressant, le peptide biologiquement actif correspond à la séquence SEQ ID n°2.

Des formes homologues de ces séquences sont également décrites. Le terme « homologue » désigne, toute séquence peptidique identique à au moins 50 %, ou de préférence au moins 80 %, et encore plus préférentiellement à au moins 90 % de ladite séquence peptidique, choisie parmi les séquences SEQ ID n° 1 à SEQ ID n° 4. Par « séquence peptidique identique à au moins X % », on entend désigner un pourcentage d'identité entre les résidus d'acides aminés des deux séquences à comparer, obtenu après l'alignement optimal des deux séquences. L'alignement optimal est obtenu à l'aide d'algorithmes d'homologies locales tels que ceux utilisés par les logiciels informatiques BLAST P ou T BLAST N disponibles sur le site NCBI.

Le terme « homologue » peut également désigner un peptide qui diffère de la séquence d'un peptide de séquence SEQ ID n° 1 à SEQ ID n° 4 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et Ile ; entre Ser et Thr ; entre les résidus acides Asp et Glu ; entre Asn et Gln ; et entre les résidus basiques Lys et Arg ; ou entre les résidus aromatiques Phe et Tyr.

Le terme « acide aminé » se réfère ici à tout acide organique naturel ou non naturel ayant la formule :

-NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme « alkyl », on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Le terme « peptide » désigne un enchaînement de deux ou plusieurs acides aminés liés entre eux par des liaisons peptidiques ou par des liaisons peptidiques modifiées, le terme « polypeptide » désignant un peptide de taille plus importante.

Par « peptide », il faut entendre le peptide naturel ou synthétique de l'invention tel que décrit ci-dessus ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit. De façon à améliorer la résistance à la dégradation, il peut être nécessaire d'utiliser une forme protégée du peptide. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées. Ainsi, l'invention concerne une composition telle que définie précédemment, caractérisée par le fait que le peptide de formule générale (I) possède au moins un groupement fonctionnel protégé par un groupement protecteur, ce groupement protecteur étant soit une acylation ou une acétylation de l'extrémité amino-terminale, soit une amidation ou une estérification de l'extrémité carboxy-terminale, soit les deux. L'extrémité amino-terminale peut être protégée par un groupement acétyle, un groupement benzoyle, un groupement tosyle ou un groupement benzyloxycarbonyle. De préférence, on utilise une protection basée sur l'amidation de la fonction hydroxyle de l'extrémité carboxy-terminale par un groupement NYY avec Y représentant une chaîne alkyle de C₁ à C₄, ou l'estérification par un groupement alkyle. Il est également possible de protéger les deux extrémités du peptide.

Les dérivés de peptides concernent aussi les acides aminés et les peptides reliés entre eux par une liaison pseudo-peptidique. On entend par « liaison pseudo-peptidique », tous les types de liaisons susceptibles de remplacer les liaisons peptidiques « classiques ». Dans le domaine des acides aminés, la géométrie des molécules est telle qu'elles peuvent théoriquement se présenter sous la forme d'isomères optiques différents. Il existe, en effet, une conformation moléculaire de l'acide aminé (AA) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa). Les acides aminés naturels sont toujours de conformation lévogyre, en conséquence, un peptide d'origine naturelle ne sera constitué que d'acides aminés de type L-aa. Cependant, la synthèse chimique en laboratoire permet de préparer des acides aminés ayant les deux conformations possibles. A partir de ce matériel de base, il est ainsi possible d'incorporer lors de la synthèse de peptide aussi bien des acides aminés sous forme d'isomères optiques dextrogyre ou lévogyre. Ainsi, les acides aminés constituant le peptide selon l'invention peuvent être sous configuration Let D- ; de manière préférentielle, les acides aminés sont sous forme L. Le peptide peut donc être sous forme L-, D- ou DL-.

Le peptide de formule générale (I) peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide peut également être obtenu par fermentation d'une souche de bactéries modifiées ou non, par génie génétique, ou encore par extraction de protéines d'origine animale ou végétale, préférentiellement d'origine végétale, suivie d'une hydrolyse contrôlée qui libère des fragments peptidiques correspondant totalement ou partiellement aux peptides de formule générale (I).

De très nombreuses protéines trouvées dans les plantes sont susceptibles de contenir ces séquences au sein de leur structure. L'hydrolyse ménagée permet de dégager ces fragments peptidiques. Il est possible, mais non nécessaire pour réaliser l'invention, d'extraire soit les protéines concernées d'abord et de les hydrolyser ensuite, soit d'effectuer l'hydrolyse d'abord sur un extrait brut et de purifier les fragments peptidiques ensuite. Il est également possible d'utiliser certains extraits hydrolysés sans en purifier les fragments peptidiques correspondant aux peptides de formule générale 1 selon l'invention, mais en s'assurant toutefois de la présence desdits fragments par des moyens analytiques appropriés.

D'autres procédés plus simples ou plus complexes peuvent être envisagés par l'homme du métier connaissant le métier de synthèse, d'extraction et de purification des protéines et des peptides. Ainsi le peptide selon l'invention peut être d'origine naturelle ou synthétique. Préférentiellement selon l'invention, le peptide est obtenu par synthèse chimique.

Le principe actif peut être un mélange de dérivés peptidiques et/ou constitués de dérivés d'acides aminés.

Selon un mode de réalisation avantageux de l'invention, le principe actif est préalablement solubilisé dans un ou plusieurs solvants classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, le principe actif est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans, ou fixé sur, tout vecteur cosmétiquement ou pharmaceutiquement acceptable.

La composition utilisable selon l'invention peut en particulier consister en une composition pour soins capillaires, et notamment un shampooing, un après-shampooing, une lotion de mise en plis, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non suivie d'un rinçage, ou encore sous forme de shampooing.

Elle peut également se présenter sous forme de teinture ou de mascara à appliquer au pinceau ou au peigne, en particulier sur les cils, les sourcils ou les cheveux.

Il est bien entendu que le principe actif peut être utilisé seul ou bien en association avec au moins un autre principe actif, dans une composition cosmétique ou pour la préparation d'une composition pharmaceutique et/ou dermatologique.

Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention se présentent sous une forme adaptée à l'application par voie topique. Ces compositions doivent donc contenir un milieu cosmétiquement et/ou dermatologiquement acceptable, c'est-à-dire compatible avec la peau et les phanères, et couvrent toutes les formes cosmétiques ou dermatologiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, adaptés à une application sur la peau, les lèvres et/ou les phanères.

Ces compositions comprennent les excipients nécessaires à leur formulation, tels que solvants, épaississants, diluants, tensioactifs, anti-oxydants, colorants, conservateurs, parfums.

Avantageusement, les compositions utilisables contiennent au moins un autre agent actif favorisant l'action des peptides selon l'invention. Ainsi, la composition selon l'invention peut associer, au principe actif selon l'invention, des agents actifs ayant une action antioxydante, ou encore stimulant la synthèse des macromolécules dermiques, ou encore stimulant le métabolisme énergétique. Par exemple, en tant qu'agents actifs ayant une action anti-radicalaire ou antioxydante, on peut citer la vitamine C, la vitamine E, le coenzyme Q10 et les extraits polyphénoliques de plantes.

On peut également citer en tant qu'agents actifs stimulant les synthèses des macromolécules dermiques (laminine, fibronectine, collagène), par exemple le peptide de collagène commercialisé sous le nom « Collaxyl® » par la société Vincience.

Enfin en tant qu'agents actifs stimulant le métabolisme énergétique, on peut citer le principe actif commercialisé sous la dénomination « GP4G® » par la société Vincience.

Selon un autre aspect, la composition selon l'invention peut être une composition solaire, c'est-à-dire une composition aidant à la protection contre le rayonnement solaire. Ainsi, il peut être avantageusement ajouté, à la composition selon l'invention, des actifs aidant à la protection solaire tels que, par exemple, des filtres solaires.

Il est bien évident que l'invention s'adresse aux mammifères en général, et plus particulièrement aux êtres humains.

La quantité efficace de principe actif correspond à la quantité nécessaire afin d'obtenir le résultat recherché, à savoir, activer le cytochrome c, et augmenter le niveau énergétique cellulaire, et plus généralement, protéger la peau et les phanères des agressions extérieures et lutter contre le vieillissement cutané

Selon un mode de réalisation avantageux de l'invention, le principe actif est présent dans les compositions de l'invention à une concentration comprise entre 0,0005 et 500 ppm (parties par million) environ, et préférentiellement à une concentration comprise entre 0,01 et 5 ppm environ par rapport au poids total de la composition finale.

Ces compositions pourront notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Par ses activités particulières, le principe actif selon l'invention pourra être utilisé avantageusement dans une composition cosmétique ou pour la préparation d'une composition pharmaceutique.

Notament, l'invention porte sur une composition comprenant le principe actif pour son utilisation dans un traitement dermatologique

ou pour prévenir ou traiter les dommages causés à la peau et aux phanines par les rayonnements UV.

En particulier, le principe actif pourra être utilisé avantageusement dans une composition cosmétique destinée à lutter de manière préventive et/ou curative contre les manifestations du vieillissement cutané et, plus spécifiquement, afin de lutter contre et/ou de prévenir le vieillissement photo-induit (photo-vieillissement). Par manifestations cutanées du vieillissement, on entend toutes modifications de l'aspect extérieur de la peau et des phanères dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, la décoloration des cheveux ou les taches sur les ongles, mais également toute modification interne de la peau qui ne se traduit pas systématiquement par un aspect extérieur modifié comme, par exemple, toute dégradation interne de la peau consécutive à une exposition aux rayonnements ultraviolets (UV). Le principe actif, ou la composition le contenant, permettra de lutter, en particulier, contre la perte d'élasticité et de fermeté de la peau.

Le principe actif, peut être utilisé avantageusement dans une composition cosmétique pour protéger la peau et les phanères contre tous types d'agressions extérieures. L'utilisation du principe actif, ou d'une composition le contenant, va permettre à la peau et aux phanères d'être protégés et de mieux résister aux stress environnementaux.

On entend par l'expression « agressions extérieures », les agressions que peuvent produire l'environnement. A titre d'exemple, on peut citer des agressions telles que la pollution, les UV, ou encore les produits à caractère irritant tels que les tensioactifs, les conservateurs ou les parfums. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehyde), ou bien encore la fumée de cigarette.

Le principe actif peut être avantageusement utilisé dans une composition cosmétique ou pour la préparation d'une composition pharmaceutique, en tant qu'agent photo-protecteur et, plus particulièrement, en tant qu'agent photo-protecteur dit « secondaire ». On distingue, en effet, les agents photo-protecteurs primaires des agents photo-protecteurs secondaires. Les agents photo-protecteurs primaires sont des substances qui exercent un pouvoir physique : ils sont en mesure d'absorber les rayonnements UV et de les restituer sous forme de chaleur afin de protéger la peau. Les agents photo-protecteurs secondaires sont des substances qui ont généralement un effet biologique ; ce sont, par exemple, les agents capables de limiter les dommages causés à l'ADN et aux membranes par la pénétration des rayonnements UV dans la peau.

L'utilisation dans une composition cosmétique d'une quantité efficace de principe actif l'invention pour augmenter la synthèse d'ATP intracellulaire des cellules de la peau est décrite.

L'utilisation dans une composition cosmétique d'une quantité efficace de principe actif selon l'invention, pour prévenir les dommages causés à la peau par une exposition au soleil ou une exposition à des rayonnements ionisants lors de radiothérapies est décrite.

L'utilisation dans une composition cosmétique, d'une quantité efficace de principe actif selon l'invention pour stimuler les mitochondries, en particulier sur les zones du corps exposées aux rayonnements UV est également décrite.

L'utilisation dans une composition cosmétique, d'une quantité efficace de principe actif tel que décrit précédemment pour protéger la peau des dommages causés par les radicaux libres est également décrite.

En outre, l'utilisation d'une quantité efficace de principe actif, tel que défini précédemment, pour la préparation d'une composition pharmaceutique destinée à prévenir ou à lutter contre les pathologies liées à des dysfonctionnements mitochondriaux, tels que certaines dégénérescences neuromusculaires ou cardiaques, le diabète de type II ou certaines pathologies du vieillissement est également décrite.

Engin, un procédé de traitement cosmétique destiné à stimuler les défenses et à protéger la peau et les phanères des agressions extérieures et à lutter contre le vieillissement cutané, caractérisé par l'application sur la peau ou les phanères à traiter, d'une composition contenant une quantité efficace de principe actif est également décrit.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.

### Exemple 1 : Mise en évidence de l'effet stimulant du peptide SEQ ID n'2 sur la synthèse d'ATP intracellulaire

Le but de cette étude est de déterminer l'influence du peptide SEQ ID n°2 sur la synthèse d'ATP, produit par les mitochondries.

Protocole : Cette étude s'effectue à l'aide d'un Kit « ATP Bioluminescence Assay Kit HS Il » (Roche Applied Science). Des fibroblastes dermiques sont traités avec une solution à 1 %, d'une solution à 50 ppm, contenant le peptide SEQ ID n°2, représentatif de la famille de peptide selon l'invention, pendant une période allant de 1 à 3 heures. A la fin de l'incubation, les puits sont rincés avec 2 ml de PBS froid avant d'ajouter 250 µl d'un tampon de lyse fournis par le kit. Les cellules de chaque puits sont ensuite grattées puis récoltées dans des tubes de 14 ml. Chaque puits est rincé avec 2 x 500 µl PBS froid et le tout est de nouveau récolté dans les tubes respectifs. A partir de ces échantillons, une dilution est réalisée au 1/12000^{ième} dans du PBS froid avant chaque lecture. Le dosage d'ATP est réalisé sur ces échantillons : 50 µL de cette dilution sont déposés dans une luma-cuvette et 50 µL de luminol sont ajoutés. Après 10 secondes, la lecture de la luminescence est déclenchée. Les valeurs sont standardisées par rapport à la quantité de protéines pour chaque échantillon. Les mesures sont effectuées à l'aide d'un appareil : le Biocounter M2010A LUMAC®/3M.

Résultats : Les dosages d'ATP montrent qu'il y a une augmentation de la quantité d'ATP intracellulaire de 41 % après 1 heure et de 27 % après 3 heures, dans des cellules traitées par le peptide SEQ ID n°5, en comparaison des cellules non traitées.

Conclusion : Le peptide SEQ ID n°2 augmente significativement le niveau énergétique de cellules cutanées telles que les fibroblastes, et plus généralement stimule les fonctions mitochondriales.

### Exemple 2 : Mise en évidence de l'effet activateur du peptide SEQ ID n'2 sur l'expression de cytochrome c

Le but de cette étude est de déterminer l'influence du peptide SEQ ID n°2 sur l'expression du cytochrome c. Pour cela, la quantité de cytochrome c a été évaluée par la technique de l'immuno-transfert (ou western blot).

Protocole : Des fibroblastes dermiques humains sont traités avec une solution à 1 %, d'une solution mère à 50 ppm de peptide SEQ ID n°2, pendant 72 heures. Les cellules sont ensuite lysées et homogénéisées par sonication, puis centrifugées 10 minutes à 1000 g. Les échantillons, standardisés pour leur concentration en protéine (dosage kit BCA , Pierce), sont soumis à une électrophorèse sur gel Bis-TRIS 4-12 % (InvitroGen). Après électro-transfert, les membranes sont incubées pendant une nuit avec un anticorps anti-cytochrome c, dilué au 1/500 (Mouse monoclonal anti cytochrome c, TEBU). Un anticorps secondaire, couplé à la peroxydase et dilué au 1/5000, est ensuite utilisé (peroxydase conjugated F(ab')₂, Goat antimouse, Immunotech). Le signal chimioluminescent est ensuite quantifié à l'aide du kit Supersignal West Femto Trial kit et lu dans une chambre de lecture (Multilmage light Cabinet, Alpha Immunotech Corporation).

Résultats : On observe une nette augmentation de l'expression du cytochrome c dans les fibroblastes traités par le peptide SEQ ID n°2.

Conclusions : Le peptide SEQ ID n°2, à la concentration de 0,05 ppm, stimule fortement l'expression du cytochrome c dans les cellules cutanées, et plus généralement les fonctions mitochondriales.

### Exemple 3 : Mise en évidence de l'effet activateur du peptide SEQ ID n°2 sur l'activité enzymatique de la cytochrome oxydase

Le but de cette étude est de déterminer l'influence du peptide SEQ ID n°2 sur l'activité mitochondriale. Pour cela, l'activité enzymatique totale (mitochondriale) de la cytochrome oxydase a été mesurée.

Protocole : Des fibroblastes humains normaux sont traités avec une solution à 1 %, d'une solution à 50 ppm, contenant le peptide SEQ ID n°2, représentatif de la famille de peptide selon l'invention, pendant 3 heures et 24 heures. Les cellules sont récoltées, rincées puis lysées par sonication. Une première centrifugation est réalisée pour éliminer les principaux débris cellulaires et le surnageant est ensuite centrifugé de nouveau à 10 000 g pendant 10 min. L'activité enzymatique est dosée dans le surnageant et/ou dans le 2^{eme} culot, par une méthode biochimique, à l'aide de kit Cytocox 1 (Sigma), puis standardisée pour la teneur en protéines (dosée par le kit BCA, Pierce).

Résultats : Les dosages montrent qu'il y a une augmentation de l'activité enzymatique de la cytochrome oxydase de 150 % après 3 heures et de 750 % après 24 heures d'application du peptide SEQ ID n°2, en comparaison des cellules non traitées.

Conclusion : Le peptide SEQ ID n°2, à la concentration de 0,05 ppm, stimule fortement l'activité enzymatique de la cytochrome oxydase, et plus généralement l'activité mitochondriale, dans les cellules cutanées.

### Exemple 4 : Mise en évidence de l'effet protecteur du peptide SEQ ID n°2 sur le potentiel de membrane mitochondriale

Le but de cette étude est de déterminer l'effet protecteur du peptide SEQ ID n°2 vis-à-vis des mitochondries de fibroblastes dermiques soumis à un stress oxydatif, provoqué par de l'eau oxygénée (H₂O₂) ou à une irradiation UVB. On utilise pour cela un marqueur du potentiel de membrane des mitochondries (JC-1). Le JC-1 est un marqueur qui émet une fluorescence différente selon le niveau de polarisation de la membrane mitochondriale.

Protocole : Les fibroblastes dermiques humains sont traités avec une solution à 1 %, d'une solution mère à 50 ppm de peptide SEQ ID n°2, pendant 96 heures, puis soumis à un stress oxydatif, provoqué par de l'H₂O₂ à 2 mM pendant 30 minutes, ou à une irradiation par des UVB à 50 mJ/cm². Des contrôles non traités par le peptide ou par de l'H₂O₂ ou non irradiés sont réalisés dans les mêmes conditions. A la fin de l'expérimentation, les cellules sont lavées, fixées et soumises à un marquage par une solution de JC-1 (Molecular Probes) à 0,2 µg/ml, afin de révéler le potentiel de membrane des mitochondries.

Résultats : Dans les fibroblastes traités par le peptide SEQ ID n°2, les mitochondries présentent une fluorescence rouge (JC-1 agrégé), signe d'un potentiel de membrane élevé, plus important que dans les cellules contrôle. Les fibroblastes irradiés ou soumis à un stress oxydatif ont des mitochondries qui fluorescent peu dans le rouge, et principalement dans le vert (JC-1 monomérique), signe d'une altération du potentiel de membrane mitochondriale. Dans ces dernières conditions, l'application du peptide SEQ ID n°2 permet d'observer une fluorescence rouge plus marquée.

Conclusions : L'application du peptide SEQ ID n°2 provoque une augmentation du potentiel de membrane des mitochondries. D'autre part, le peptide SEQ ID n°2 protège efficacement les mitochondries des cellules cutanées soumises à un stress oxydatif ou à une irradiation par des UVB.

### Exemple 5 : Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TR1 | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |

| Noms commerciaux | Noms INCI | % massique |
|---|---|---|
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n° 2 | | 3 ppm |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

### 2 -Lait après-soleil :

| Noms commerciaux | Noms INCI | % massique |
|---|---|---|
| PHASE A | | |
| Montanov L | C14-22 Alcohols (and) C12-20 Alkyl Glucoside | 3,00 |
| Waglinol 2559 | Cetearyl Isononanoate | 4,00 |
| Tegosoft TN | C12-15 Alkyl Benzoate | 3,00 |
| Huile de Noyaux d'Abricot | Prunus Armeniaca (Apricot) Kernel Oil | 2,00 |
| Huile d'Avocat | Persea Gratissima (Avocado) Oil | 1,00 |
| Abil 350 | Dimethicone | 1,00 |

| PHASE B | | |
|---|---|---|
| Eau déminéralisée | Aqua (Water) | qsp |
| | PHASE C | |
| Simulgel EG | Sodium Acrylate/Acryloyldimethyl Taurate Copolymer (and) Isohexadecane (and) Polysorbate 80 Copolymer (and) Polysorbate 80 | 0,4 |

| PHASE D | | |
|---|---|---|
| Phenonip | Phenoxyethanol (and) Methylparaben (and) Ethylparaben (and) Butylparaben (and) Propylparaben (and) Isobutylparaben Ethylparaben and Propylparaben and Buthylparaben | 0,30 |
| Germall 115 | Imidazolidinyl Urea | 0,20 |

| PHASE E | | |
|---|---|---|
| Peptide SEQ ID n° 2 | | 0,1 ppm |

Préparer la phase A sous agitation. Incorporer la gomme xanthane progressivement, sous agitation défloculeuse. Les phases C et D seront incorporées une fois le gel terminé. La phase E, préparée préalablement jusqu'à parfaite dissolution de la DHA, sera rajoutée ensuite. Ajuster le pH si nécessaire à 4 - 4,5. Colorer et parfumer.

### 3 -Crème anti-âge :

| ***Noms commerciaux*** | ***Noms INCI*** | **% *massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Pareil (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n°2 | | 0,5 ppm |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| ***Noms commerciaux*** | ***Noms INCI*** | **% *massique*** |
| Parfum | Parfum (Fragrance) | qsp |
| Colorant | | qsp |

Préparer et fondre la phase A à 65-70°C. Chauffer la phase C à 65-70°C. La phase B est ajoutée à la phase A juste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors additionnée si souhaité.

### 4 -Crème protectrice de jour:

| ***Noms commerciaux*** | ***Noms INCI*** | **% *massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Peptide SEQ ID n°2 | | 5 ppm |

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E. Le refroidissement est poursuivi sous agitation légère et la phase F rajoutée.

## Revendications

1. Composition contenant en tant que principe actif, dans un milieu physiologiquement acceptable, au moins un peptide capable d'activer le cytochrome c comprenant de 3 à 13 résidus d'acides aminés et dont la séquence répond à la formule générale (I) :
R₁-(AA)ₙ- X₁-X₂-X₃- X₄-X₅- (AA)ₚ-R₂
dans laquelle
X₁ est la tyrosine,
X₂ est la leucine,
X₃ est la lysine,
X₄ est la thréonine, la sérine, la lysine, l'arginine ou aucun acide aminé,
X₅ est la valine, l'alanine, la tyrosine, la méthionine ou aucun acide aminé AA représente un acide aminé quelconque, ou un de ses dérivés, et n et p sont des nombres entiers compris entre 0 et 4,
R₁ représente la fonction amine primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substitué par un groupement protecteur pouvant être choisi parmi une chaîne alkyle de C1 à C20, ou un groupement NH2, NHY ou NYY avec Y représentant une chaîne alkyle de C 1 à C4,
**caractérisée en ce que** ledit peptide de formule générale (I) correspond à une des séquences suivantes :
(SEQ ID n°1) Tyr-Leu-Lys-Lys-Ala.
(SEQ ID n°2) Tyr-Leu-Lys-Lys-Ala -NH₂
(SEQ ID n°3) Tyr-Leu-Lys-Lys-NH₂
(SEQ ID n°4) Tyr-Leu-Lys.

2. Composition selon la revendication 1 **caractérisée en ce que** le peptide de formule générale (I) correspond à la séquence SEQ ID n° 1.

3. Composition selon la revendication 1 **caractérisée en ce que** le peptide de formule générale (I) correspond à la séquence SEQ ID n°2.

4. Composition selon l'une quelconque des revendications précédentes, **caractérisée en ce que** le peptide de formule générale (I) possède au moins un groupement fonctionnel protégé par un groupement protecteur, ce groupement protecteur étant soit une acylation ou une acétylation de l'extrémité amino-terminale, soit une amidation ou une estérification de l'extrémité carboxy-terminale, soit les deux.

5. Composition selon l'une quelconque des revendications précédentes, **caractérisée en ce que** le principe actif est présent dans la composition à une concentration comprise entre 0,0005 et 500 ppm environ par rapport au poids total de la composition.

6. Composition selon la revendication 5, **caractérisée en ce que** le principe actif est présent dans la composition à une concentration comprise entre 0,01 et 5 ppm par rapport au poids total de la composition.

7. Composition selon l'une quelconque des revendications précédentes, **caractérisée en ce que** le principe actif, est préalablement solubilisé dans un ou plusieurs solvants choisis parmi l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

8. Composition selon l'une quelconque des revendications précédentes, **caractérisée en ce qu'**elle se présente sous une forme adaptée à l'application par voie topique.

9. Composition selon l'une quelconque des revendications précédentes, **caractérisée en ce qu'**elle contient, en outre, au moins un autre agent actif choisi parmi la vitamine C, la vitamine E, le coenzyme Q10, les extraits polyphénoliques de plantes, le peptide de collagène commercialisé sous le nom de « Collaxyl ® » ou le principe actif commercialisé sous la dénomination « GP4G® ».

10. Composition comprenant un principe actif tel que défini dans l'une quelconque des revendications 1 à 4, pour son utilisation dans un traitement dermatologique.

11. Composition comprenant un principe actif tel que défini dans l'une quelconque des revendications 1 à 4 pour prévenir ou traiter les dommages causés à la peau et aux phanères par les rayonnements UV.

12. Utilisation cosmétique, non-thérapeutique, de la composition comprenant un principe actif tel que défini dans l'une quelconque des revendications 1 à 4 pour prévenir ou traiter les signes cutanés du vieillissement et/ou du photo-vieillissement.

## Claims

1. Composition containing as the active substance thereof, in a physiologically acceptable medium, at least one peptide capable of activating cytochrome c comprising 3 to 13 amino acid residues and wherein the sequence complies with the general formula (I):
R₁-(AA)ₙ-X₁-X₂-X₃-X₄-X₅-(AA)ₚ-R₂
wherein
X₁ is tyrosine,
X₂ is leucine,
X₃ is lysine,
X₄ is threonine, serine, lysine, arginine or no amino acid,
X₅ is valine, alanine, tyrosine, methionine or no amino acid
AA represents any amino acid, or any of the derivatives thereof, and n and p are integers between 0 and 4,
R₁ represents the primary amine function of the N-terminal amino acid, which is free or substituted by a protecting group that may be chosen from an acetyl group, a benzoyl group, a tosyl group or a benzyloxycarbonyl group,
R₂ represents the hydroxyl group of the carboxyl function of the C-terminal amino acid, which is free or substituted by a protecting group that may be chosen from a C1 to C20 alkyl chain, or an NH2, NHY or NYY group where Y represents a C1 to C4 alkyl chain,
**characterised in that** said peptide having general formula (I) corresponds to the following sequences:
(SEQ ID No. 1) Tyr-Leu-Lys-Lys-Ala
(SEQ ID No. 2) Tyr-Leu-Lys-Lys-Ala-NH₂
(SEQ ID No. 3) Tyr-Leu-Lys-Lys-NH₂
(SEQ ID No. 4) Tyr-Leu-Lys.

2. Composition according to claim 1 **characterised in that** the peptide having general formula (I) corresponds to the sequence SEQ ID No. 1.

3. Composition according to claim 1 **characterised in that** the peptide having general formula (I) corresponds to the sequence SEQ ID No. 2.

4. Composition according to any of the above claims, **characterised in that** the peptide having general formula (I) has at least one functional group protected by a protecting group, said protecting group being either an acylation or acetylation of the amino-terminus, or an amidation or esterification of the carboxy-terminus, or both.

5. Composition according to any of the above claims, **characterised in that** the active substance is present in the composition at a concentration between approximately 0.0005 and 500 ppm with respect to the total weight of the composition.

6. Composition according to claim 5, **characterised in that** the active substance is present in the composition at a concentration between 0.01 and 5 ppm with respect to the total weight of the composition.

7. Composition according to any of the above claims, **characterised in that** the active substance is previously solubilised in one or a plurality of solvents chosen from water, glycerol, ethanol, propylene glycol, butylene glycol, dipropylene glycol, diglycol ethoxylates or propoxylates, cyclic polyols, petroleum jelly, a plant oil or any mixture of these solvents.

8. Composition according to any of the above claims, **characterised in that** it is in a form suitable for topical application.

9. Composition according to any of the above claims, **characterised in that** it further contains at least one other active agent chosen from vitamin C, vitamin E, coenzyme Q10, plant polyphenol extracts, collagen peptide marketed under the brand "Collaxyl ®" or the active substance marketed under the brand "GP4G®".

10. Composition comprising an active substance as defined in any of claims 1 to 4, for the use thereof in a dermatological treatment.

11. Composition comprising an active substance as defined in any of claims 1 to 4 for preventing or treating damage caused to skin and appendages by UV rays.

12. Non-therapeutic, cosmetic use of the composition comprising an active substance as defined in any of claims 1 to 4 for preventing or treating the skin signs of ageing and/or photo-ageing.

## Patentansprüche

1. Verbindung, die als Wirkstoff in einem physiologisch akzeptablen Medium mindestens ein Peptid umfasst, das dazu in der Lage ist, das Cytochrom c zu aktivieren, das von 3 bis 13 Aminosäurerückstände umfasst, und dessen Sequenz der folgenden allgemeinen Formel (I) entspricht:
R₁- (AA)ₙ-X₁-X₂-X₃-X₄-X₅- (AA) p-R₂
wobei:
X₁ Tyrosin ist,
X₂ Leucin ist,
X₃ Lysin ist,
X₄ Threonin, Serin, Lysin, Arginin oder keine Aminosäure ist,
X₅ Valin, Alanin, Tyrosin, Methionin oder keine Aminosäure ist,
AA irgendeine Aminosäure oder eines seiner Derivate darstellt, und n und p ganze Zahlen zwischen 0 und 4 sind,
R₁ die primäre Amionfunktion der N-terminalen Aminosäure darstellt, frei oder substituiert durch eine Schutzgruppe,
die aus einer Acetylgruppe, einer Benzoylgruppe, einer Tosylgruppe oder einer Benzyloxycarbonylgruppe gewählt werden kann,
R₂ die Hydroxylgruppe der Carboxylfunktion der C-terminalen Aminosäure darstellt, frei oder substituiert durch eine Schutzgruppe, die aus einer Alkylkette von C1 bis C20 oder einer NH2-, NHY- oder NYY-Gruppe gewählt werden kann, wobei Y eine Alkylkette von C1 bis C4 darstellt,
**dadurch gekennzeichnet, dass** des Peptid mit der allgemeinen Formel (I) einer der folgenden Sequenzen entspricht:
(SEQ ID n°1) Tyr-Leu-Lys-Lys-Ala.
(SEQ ID n°2) Tyr-Leu-Lys-Lys-Ala -NH₂
(SEQ ID n°3) Tyr-Leu-Lys-Lys-NH₂
(SEQ ID n°4) Tyr-Leu-Lys.

2. Verbindung nach Anspruch 1, **dadurch gekennzeichnet, dass** das Peptid mit der allgemeinen Formel (I) der Sequenz SEQ ID n°1 entspricht.

3. Verbindung nach Anspruch 1, **dadurch gekennzeichnet, dass** das Peptid mit der allgemeinen Formel (I) der Sequenz SEQ ID n°2 entspricht.

4. Verbindung nach einem der vorhergehenden Ansprüche, **dadurch gekennzeichnet, dass** das Peptid mit der allgemeinen Formel (I) mindestens eine funktionelle Gruppe aufweist, die von einer Schutzgruppe geschützt ist, wobei diese Schutzgruppe entweder eine Acylierung oder eine Acetylierung des amino-terminalen Endes oder eine Amidbildung oder eine Veresterung des carboxy-terminalen Endes oder beides ist.

5. Verbindung nach einem der vorhergehenden Ansprüche, **dadurch gekennzeichnet, dass** der Wirkstoff in der Verbindung in einer Konzentration zwischen ungefähr 0,0005 und 500 ppm mit Bezug auf das Gesamtgewicht der Verbindung vorhanden ist.

6. Verbindung nach Anspruch 5, **dadurch gekennzeichnet, dass** der Wirkstoff in der Verbindung in einer Konzentration zwischen ungefähr 0,01 und 5 ppm mit Bezug auf das Gesamtgewicht der Verbindung vorhanden ist.

7. Verbindung nach einem der vorhergehenden Ansprüche, **dadurch gekennzeichnet, dass** der Wirkstoff im Voraus in einem oder in mehreren Lösemitteln solubilisiert wird, ausgewählt aus Wasser, Glycerol, Ethanol, Propylenglycol, Butylenglycol, Dipropylinglycol, ethoxylierte oder propoxylierte Diglykolen, cyclischen Polyolen, Vaselin, einem pflanzlichen Öl oder jeder Mischung dieser Lösemittel.

8. Verbindung nach einem der vorhergehenden Ansprüche, **dadurch gekennzeichnet, dass** sie eine Form aufweist, die für die Anwendung auf topischem Weg ausgelegt ist.

9. Verbindung nach einem der vorhergehenden Ansprüche, **dadurch gekennzeichnet, dass** sie außerdem mindestens einen weiteren Wirkstoff umfasst, ausgewählt aus Vitamin C, Vitamin E, dem Coenzym Q10, den Polyphenolextrakten von Pflanzen, dem Collagenpeptid, das unter dem Namen "Collaxyl" vertrieben wird, oder dem Wirkstoff, der unter der Bezeichnung "GP4G®" vertrieben wird.

10. Verbindung, umfassend einen Wirkstoff wie in einem der Ansprüche 1 bis 4 definiert, für seine Verwendung bei einer dermatologischen Behandlung.

11. Verbindung, umfassend einen Wirkstoff wie in einem der Ansprüche 1 bis 4 definiert, um Beschädigungen vorzubeugen oder diese zu behandeln, die von den UV-Strahlen an der Haut und an den Hautanhangsgebilden verursacht wurden.

12. Kosmetische, nicht therapeutische Verwendung der Verbindung, umfassend einen Wirkstoff, wie in einem der Ansprüche 1 bis 4 definiert, um den Hautzeichen der Alterung und/oder der Lichtalterung vorzubeugen oder diese zu behandeln.
